# EUROPÄISCHE PATENTSCHRIFT

(11) **EP 0 418 391 B1**
(45) Veröffentlichungstag und Bekanntmachung des Hinweises auf die Patenterteilung: **11.10.1995**
(21) Anmeldenummer: 90900429.3
(22) Anmeldetag: 06.06.1989
(51) Int. Cl.: A61B 17/24, A61M 25/00

(54) **ANORDNUNG ZUR BEHANDLUNG VON SINUSITIS**
DEVICE FOR TREATMENT OF SINUSITIS
DISPOSITIF DE TRAITEMENT DE LA SINUSITE

(30) Priorität: 29.03.1989 SU 4664121
(43) Veröffentlichungstag der Anmeldung: 27.03.1991
(73) Patentinhaber: YAROSLAVSKY MEZHOTRASLEVOI NAUCHNO-TEKHNICHESKY TSENTR, Yaroslavl, 150000 (SU)
(72) Erfinder: KOZLOV, Vladimir Sergeevich, Yaroslavl, 150000 (SU)
(74) Vertreter: Füchsle, Klaus, Dipl.-Ing.
(86) Internationale Anmeldenummer: SU8900157
(87) Internationale Veröffentlichungsnummer: WO9011053

(56) Entgegenhaltungen:
- EP-A- 0 080 436
- GB-B- 1 340 788
- SU-A- 0 627 828
- SU-A- 0 683 756
- SU-A- 1 311 714
- US-A- 4 714 460

## Beschreibung

### Gebiet der Technik

Die vorliegende Erfindung betrifft die Medizin und bezieht sich insbesondere auf eine Einrichtung zur Behandlung von Sinusitiden.

### Vorhergehender Stand der Technik

Es ist eine Einrichtung zur Behandlung von Sinusitiden (Nasennebenhöhlenentzündungen) bekannt, welche ein längliches rohrförmiges Gehäuse mit zwei Längskanälen enthält. An diesem Gehäuse ist zwischen dessen Enden eine aufblasbare Manschette angebracht, während an einem der Enden des Gehäuses eine aufblasbare Ballonflasche eingebaut ist. Im Gehäuse sind zwei Längskanäle vorgesehen, von denen der erste für die Luftzufuhr in die aufblasbare Ballonflasche, der zweite aber für die Absaugung eines pathologischen Inhaltes und für die Verabreichung von Präparaten bestimmt sind. Dieser zweite Kanal endet zwischen der Manchette und der Ballonflasche und steht über seine stirnseitige Öffnung mit der Außenfläche des Gehäuses (SU, A, 1311714) in Verbindung. Bei Verwendung einer solchen Einrichtung ordnet man die Ballonflasche und die Maschette in der Choane bzw. in dem Vorhof der Nase an und bläst unter Bildung eines geschlossenen Hohlraums auf und nimmt eine Absaugung über den zweiten Kanal vor. Dabei wird ein eitriges oder schleimigeitriges Sekret, nachdem es den mittleren Nasengang passiert hat und über die Schleimhaut gewandert ist, in die Öffnung des zweiten Kanals geleitet, verbleibt zum Teil an der Oberfläche der Schleimhaut unvermeidlich und gerät bei anschließender Einführung von Heilmittelpräparaten über den zweiten Kanal wieder in die Sinusse. Daher erweist sich in einigen Fällen die Behandlung mit Hilfe dieser Einrichtung als wenig effektiv.

### Offenbarung der Erfindung

Der vorliegenden Erfindung ist die Aufgabe zugrundegelegt, die Einrichtung zur Behandlung von Sinusitiden derart auszuführen, daß die Entfernung eines pathologischen Inhaltes, der im Nasengang verbleibt, gewährleistet wird.

Die gestellte Aufgabe wird durch die Einrichtung gemäß Anspruch 1 gelöst.

Der dritte Kanal bietet die Möglichkeit, die Nasenhöhle durch Zufuhr einer Spülflüssigkeit in die Hinterteile der Nasenhöhle zu spülen und gleichzeitig damit deren Evakuierung vorzunehmen, was eine Entfernung des Präzipitats eines an der Schleimhaut niedergeschlagenen pathologischen Inhaltes gewährleistet, wodurch die Effektivität der Behandlung von Sinusitiden erhöht wird.

### Kurzbeschreibung der Zeichungen

Im weiteren wird die Erfindung an Hand einer konkreten, aber die vorliegende Erfindung nicht begrenzenden Ausführungsform der Einrichtung zur Behandlung von Sinusitiden unter Bezugnahme auf die beigelegten Zeichnungen näher erläutert. Es zeigt:
Fig. 1 eine Ansicht der erfindungsgemäßen Einrichtung zur Behandlung von Sinusitiden im Schnitt;
Fig. 2 die Einrichtung nach Fig. 1, die aber am Patienten montiert ist.

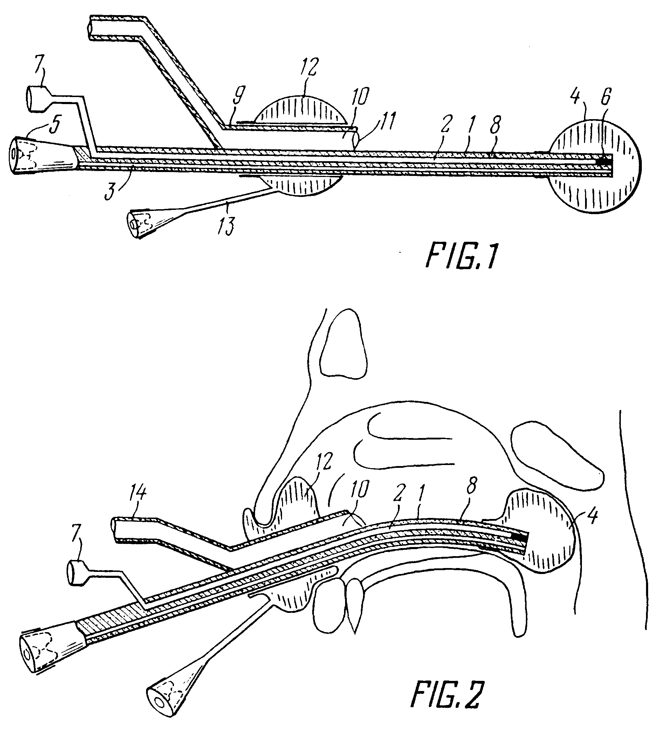

Die in Fig. 1 gezeigte Einrichtung zur Behandlung von Sinusitiden (Nasennebenhöhlenentzündungen) enthält ein längliches rohrförmiges Gehäuse 1 aus Naturkautschuk, armiert mit einem ins Innere des Gehäuses eingelegten Hohlstab oder - rohr 2 aus biegsamem Material, so daß das Gehäuse 1 eine biegsame Konstruktion darstellt. Dieser Hohlstab bildet einen Kanal.

Im Inneren des Gehäuses 1 ist ein zum Hohlstab 2 parallel verlaufender durchgehender Kanal 3 ausgeführt, der mit einer am Ende des Gehäuses 1 eingebauten aufblasbaren Ballonflasche 4, in Verbindung steht, die der Applikation in den Nasenrechenraum dient, wie dies in Fig. 2 am besten gezeigt ist. Dieser Kanal 3 ist am gegenüberliegenden Ende mit einem Übergangsstück 5 für die Einführung einer Spritze verbunden. Der Hohlstab 2 ist an dem einen Ende mit einem Stopfen verschlossen und an dem anderen Ende aus dem Gehäuse 1 herausgeführt sowie mit einem Übergangsstück 7 für eine Spritze (nicht gezeigt) versehen. In der Nähe der aufblasbaren Ballonflasche 4 ist in der Seitenwand des Gehäuses 1 eine Öffnung 8 vorgesehen, über die der Kanal des Hohlstabes 2 mit der Ausßenfläche des Gehäuses 1 in Verbindung steht. Das Gehäuse 1 hat auch einen kurzen rohrförmigen Abschnitt 9, der einen Kanal 10 mit einer stirnseitigen Öffnung 11 bildet, die mit der Außenfläche des Gehäuses 1 in Verbindung steht. Zwischen den Enden des Gehäuses 1 findet in der Zone, wo der rohrförmige Abschnitt 9 angeordnet ist, eine Manschette 12 Platz, die über ein individuelles Rohr 13 mit einer Luftquelle in Verbindung steht.

Man führt die Behandlung mit Hilfe der erfindungsgemäßen Einrichtung wie folgt durch.

Nach der Anästhesie und Anämisierung der erkrankten Nasenhälfte mit einer 2%-igen Dikainlösung unter Zusatz einer Lösung von Adrenalin (1 Tropfen der 0,1%-igen Adrenalinlösung je 1 ml der 2%-igen Dikainlösung) reinigt man die Nasenhöhle durch Ausschneuzen. Entlang des unteren Teils des gemeinsamen Nasenganges führt man die Einrichtung in die Nasenhöhle bis auf den Nasenrachenraum ein.

Dabei schleppt man die aufblasbare Ballonmanschette 4 in abgelassenem Zustand durch Verschiebung bis zur Choane durch, während man die Manschette 12 durch deren Verschiebung entlang des Gehäuses 1 in den Vorhof der Nase entsprechen den individuellen Abmessungen des gemeinsamen Nasenganges einführt. Alsdann bläst man die Ballonflasche 4 und die Manschette 12 auf, wodurch die Nasenhöhle verschlossen wird. Die entsprechenden Übergangsstücke werden nachher mit Stöpfen abgeschlossen. An das Übergangsstück 14 des Kanals 10 schließt man eine Spritze mit einer Gefäßinhalt von 20 ml oder ein Mundstück eines Elektroabsauggerätes (nicht gezeigt) an. Darauf erzeugt man in der Nasenhöhle einen Unterdruck bis auf 0,4 ... 0,6 atm, wodurch der pathologische Inhalt aus den Nasennebenhöhlen in den Unterdrucksbereich gerichtet und in die Spritze oder den Aufnehmer des Elektroabsauggeräts abgesaugt wird. Nachdem die Erzeugung eines weiteren Unterdrucks bereits zu keinem Gelangen des pathologischen Sekrets in die Spritze oder den Aufnehmer des Elektroabsauggeräts geführt hat, schließt man ans Übergangsstück 7 des Rohrs 2 eine Spritze mit einer Antiseptikumlösung an. Die Einspritzung des Antiseptikums über den Hohlstab oder das Hohlrohr 2 erfolgt gleichzeitig mit der Erzeugung eines negativen Druckes unter Zuhilfenahme des Kanals 10. Eine solche Spülung führt zur Evakuierung eines eitrigen oder schleimigen Sektrets aus der Nasenhöhle. Die Strömung der Flüssigkeit von hinten nach vorn trägt bei gleichzeitiger Erzeugung eines Unterdruckes zu einer Erhöhung der Absaugungseffektivität bei, weil hierbei dem Effekt des negativen Druckes noch eine Wirkung des Wasserstrahlabsaugung hinzugefügt wird.

Es ist zu beachten, daß man die Absaugung und die Spülung am sitzenden Kranken vornimmt, wobei man den Kopf in die Richtung neigt, die der erkrankten Seite entgegengesetzt ist.

Nachdem die Spülung abgeschlossen ist, wovon die Evakuierung der Antiseptikumslösung ohne Nebenbestandteile aus der Nasenhöhle zeugt, wird der Kranke in die liegende Lateroposition übergeführt, wobei sein Kopf zu den erkranten Sinussen hin herabhängen muß, worauf in die Sinusse in bekannter Weise ein Arzneimittel appliziert wird.

Also bietet die Einrichtung zur Behandlung von Sinusitiden die Möglichkeit, einen pathologischen Inhalt aus der Nasenhöhle völlig zu entfernen, wodurch vermieden wird, daß der pathologische Inhalt weider in die Sinusse gerät, und zu einer Erhöhung der Effektivität der Therapie einer Sinusitis beigetragen wird.

### Gewerbliche Verwertbarkeit

Durch die erfindungsgemässe Einrichtung wird es vermieden, daß der pathologische Inhalt aus der Nasenhöhle wieder in die Sinusse gerät, was zu einer Erhöhung der Effektivität der Therapie einer Sinusitis beigetragen wird.

## Patentansprüche

1. Einrichtung zur Behandlung von Sinusitiden, mit einem länglichen rohrförmigen Gehäuse (1), einem zumindest im arbeitsseitigen Teil des Gehäuses angeordneten, biegsamen Verstärkungsstab (2), einer am Gehäuse an einer zwischen dessen Enden vorgesehenen, kurzen rohrförmigen Gehäuse-Abschnitt (9) angebrachten aufblasbaren Manschette (12) und einer an einem Ende des Gehäuses eingebauten Ballonflasche (4), die mit einem ersten (3) von zwei Längskanälen (3, 10) im Gehäuse in Verbindung steht, wobei der zweite (10) dieser Kanäle zwischen der Manschette (12) und der Ballonflasche (4) endet und über seine stirnseitige Öffnung (11), die im Gehäuse-Abschnitt (9) an einer senkrecht zur Längsachse des Kanals (10) sich erstreckenden Fläche angeordnet ist, mit der Oberfläche des Gehäuses in Verbindung steht, und mit einem rohrförmigen Verbindungsteil (13) zum Aufblasen der Manschette (12),
**dadurch gekennzeichnet,**
- daß das Gehäuse (1) einen dritten Kanal (2) aufweist, der sich im wesentlichen entlang des gesamten Gehäuses erstreckt und mit der Außenfläche des Gehäuses (1) über eine in der Seitenwand des Gehäuses (1) vorgesehene Öffnung (8) in Verbindung steht, die im Abschnitt zwischen der stirnseitigen Öffnung (11) des zweiten Kanals (10) und der aufblasbaren Ballonflasche (4) angeordnet ist,
- daß der Verstärkungsstab ein Hohlstab (2) ist, der den dritten Kanal bildet,
- und daß die Manschette (12) axial verschiebbar auf dem Gehäuse-Abschnitt (9) angeordnet ist.

2. Einrichtung nach Anspruch 1, dadurch gekennzeichnet, daß die Öffnung (8) des Hohlstab-Kanals (2) in der Nähe der Ballonflasche (4) vorgesehen ist.

3. Einrichtung nach Anspruch 1, dadurch gekennzeichnet, daß der Kanal-Hohlstab (2) an seinem inneren, distalen Ende über einen Stopfen verschlossen und an seinem anderen, äußeren Ende aus dem Gehäuse herausgeführt und mit einem SpritzenÜbergangsstück (7) versehen ist.

4. Einrichtung nach Anspruch 1, dadurch gekennzeichnet, daß das rohrförmige Verbindungsteil zum Aufblasen der Manschette (12) ein individuelles Rohr (13) ist.

## Claims

1. Device for treating sinusitis, having an elongated tubular housing (1), a flexible reinforcing rod (2) disposed at least in the operating-end part of the housing, an inflatable cuff (12) mounted on the housing at a short tubular housing portion (9) provided between the ends of the housing, and a balloon bottle (4) fitted on one end of the housing and connected to a first (3) of two longitudinal channels (3, 10) in the housing, the second (10) of said channels terminating between the cuff (12) and the balloon bottle (4) and being connected by its end opening (11), which is disposed in the housing portion (9) at a surface extending at right angles to the longitudinal axis of the channel (10), to the surface of the housing, and having a tubular connection part (13) for inflating the cuff (12),
characterized in
- that the housing (1) has a third channel (2), which extends substantially along the entire housing and is connected to the outer surface of the housing (1) by an opening (8), which is provided in the side wall of the housing (1) and is disposed in the portion between the end opening (11) of the second channel (10) and the inflatable balloon bottle (4),
- that the reinforcing rod is a hollow rod (2) which forms the third channel,
- and that the cuff (12) is disposed in an axially displaceable manner on the housing portion (9).

2. Device according to claim 1, characterized in that the opening (8) of the hollow rod channel (2) is provided in the vicinity of the balloon bottle (4).

3. Device according to claim 1, characterized in that the channel hollow rod (2) is closed at its inner, distal end by a stopper and at its other, outer end is led out of the housing and provided with a syringe transition piece (7).

4. Device according to claim 1, characterized in that the tubular connection part for inflating the cuff (12) is an individual tube (13).

## Revendications

1. Dispositif de traitement des sinusites, avec un conduit tubulaire (1) allongé, un tube de renforcement (2) flexible disposé au moins dans la partie du coude située du côté où l'on travaille, une manchette (12) gonflable montée sur le conduit, en une section de conduit tubulaire (9) courte, prévue entre ses extrémités et un ballon (4) solidarisée à une extrémité du conduit, qui est relié à un premier (3) de deux canaux longitudinaux (3, 10) ménagés dans le conduit, le deuxième (10) de ces canaux s'achevant entre la manchette (12) et le ballon (4) et étant relié à la surface du conduit par son ouverture frontale (11), disposée dans la section du conduit (9) sur une surface perpendiculaire à l'axe longitudinal du canal (10), et avec une partie (13) tubulaire, de liaison, destinée au gonflage de la manchette (12), caractérisé en ce que:
- le conduit (1) présente un troisième canal (2) qui s'étend sensiblement sur toute la longueur du conduit et est relié à la surface extérieure du conduit (1) par une ouverture (8) prévue dans la paroi latérale du conduit (1), cette ouverture étant disposée dans la section se trouvant entre l'ouverture (11) frontale du deuxième canal (10) et le ballon (4) gonflable,
- en ce que le tube de renforcement est un tube creux (2) qui constitue le troisième canal,
- et en ce que la manchette (12) est disposée de façon à pouvoir se déplacer axialement sur la section de conduit (9).

2. Dispositif selon la revendication 1, caractérisé en ce que l'ouverture (8) du tube (2) est prévue à proximité du ballon (4).

3. Dispositif selon la revendication 1, caractérisé en ce que le tube (2) est obturé à son extrémité distale intérieure par un bouchon et dépasse à son autre extrémité extérieure du conduit et est pourvue d'une pièce de transition (7) pour seringue.

4. Dispositif selon la revendication 1, caractérisé en ce que la partie de liaison tubulaire destinée au gonflage de la manchette (12) est un tube (13) individuel.
